# EUROPEAN PATENT SPECIFICATION

(11) **EP 0 850 306 B1**
(45) Date of publication and mention of the grant of the patent: **25.08.2004**
(21) Application number: 96932950.7
(22) Date of filing: 03.09.1996
(51) Int. Cl.: C12N 15/53, C12N 9/02

(54) **LACCASES WITH AN ALTERED pH ACTIVITY PROFILE**
LACCASE MIT VERÄNDERTEM pH-AKTIVITÄTSPROFIL
LACCASES AYANT UN PROFIL pH D'ACTIVITE MODIFIEÄÄà LACCASES AYANT UN PROFIL D'ACTIVITE pH MODIFIE

(30) Priority: 01.09.1995 US 3142
(43) Date of publication of application: 01.07.1998
(73) Proprietor: Novozymes Biotech, Inc., Davis, CA 95616 (US)
(72) Inventor: XU, Feng, Woodland, CA 95776 (US); BERKA, Randy, M., Davis, CA 95616 (US); WAHLEITHNER, Jill, Angela, Davis, CA 95616 (US)
(74) Representative: Knudsen, Sten Lottrup
(86) International application number: PCT/US1996/014087
(87) International publication number: WO 1997/009431

(56) References cited:
- EP-A- 0 506 431
- WO-A-92/01046
- WO-A-95/07988
- WO-A-95/33836
- JOURNAL OF BIOLOGICAL CHEMISTRY, vol. 263, no. 2, 15 January 1988, MD US, pages 885-896, XP002023112 U.A.GERMANN ET AL.: "Characteristics of two allelic forms of Neurospora crassa laccase"
- JOURNAL OF BIOLOGICAL CHEMISTRY, vol. 265, no. 25, 5 September 1990, MD US, pages 15224-15230, XP002023164 Y.KOJIMANI ET AL.: "Cloning, sequence analysis, and expression of ligninolytic phenoloxidase genes of the white-rot basidiomycete Coriolus hirstus"
- CELL, vol. 76, no. 2, 28 January 1994, NA US, pages 403-410, XP002023113 C.ASKWITH ET AL.: "The FET3 gene of S.cerevisieae encodes a multicopper oxidase required for ferrous iron uptake"

## Description

### Background of the Invention

### Field of the Invention

The present invention relates to mutant laccases. More specifically, the invention relates to laccases which have been modified so as to exhibit altered pH activity profiles relative to the wild-type laccase.

### Description of the Related Art

There are currently a number of well-known blue copper oxidases which have various commercial/industrial applications. Two major classes of these enzymes are recognized: (1) the single copper proteins, which are single copper-containing, blue electron-transfer proteins such as plastocyanin, azurin, stellacyanin, amicyanin, auracyanin, cucumber basic blue, mavicyanin, rusticyanin, and umecyanin; and (2) the multi-copper oxidases, which are multiple copper-containing, blue oxidoreductases such as laccase, bilirubin oxidase, phenoxazinone synthase, ascorbate oxidase, ceruloplasmin, and nitrite reductase. The blue color of these proteins arises from the so-called Type 1 (T1) copper site.

It is an object of the present invention to provide mutants of laccases which have improved properties.

### Brief Description of the Figures

Figure 1 shows the scheme for construction of intermediate plasmid pInt2.22 and oligonucleotide-directed mutagenesis of the *Myceliophthora thermophila lcc-1* gene.
Figure 2 shows the construction of the intermediate pInt1 which contains the *Aspergillus oryzae* TAKA amylase promoter and 5'-portion of the *Myceliophthora thermophila lcc-1* coding region.
Figure 3 shows the final step in construction of pRaMB 17 and its derivatives, pRaMB17M and pRaMB17Q, which direct expression of wild-type and mutant forms of *Myceliophthora thermophila* laccase (MtL).
Figure 4 shows the construction of pBANe22T which directs expression of a mutant form of *Myceliophthora thermophila* laccase.
Figure 5 shows the pH activity profiles of the wild-type (wt) and mutant *Rhizoctonia solani* laccases (RsLs) and *Myceliophthora thermophila* laccases (MtLs): wt (―); mutant M (-----); mutant T (.....); (A), RsL with 2,2'-azinobis(3-ethylbenzthiazoline-6-sulfonic acid) (ABTS); (B), RsLs with syringaldazine (SGZ); (C), MtLs with ABTS; (D), MtLS with SGZ.
Figure 6 shows the nucleotide sequence and the deduced amino acid sequence of *Rhizoctonia solani* laccase isozyme 4 *(rsl4)* gene (SEQ ID NOS:24 and 25).
Figure 7 shows the nucleotide sequence and the deduced amino acid sequence of *Myceliophthora thermophila* laccase *lcc-1* gene (SEQ ID NOS:26 and 27).

### Summary of the Invention

The present invention relates to mutants of a *Rhizoctonia solani* laccase and a *Myceliophthora* laccase having a different pH-activity profile than the wild-type laccase, comprising a mutation selected from the group consisting of (a) a substitution of one or more amino acid residues with other amino acid residues, (b) an insertion of one or more amino acid residues and/or (c) a deletion of one or more amino acid residues, wherein the substitution, insertion or deletion is carried out in specific regions. The present invention also relates to methods of altering the pH-activity profile of a fungal laccase. The present invention also relates to nucleic acid constructs comprising a nucleic acid sequence encoding the mutants of the present invention, host cells comprising the construct of the present invention.

### Detailed Description of the Invention

The present invention relates to mutants of *Rhizoctonia solani* laccase, comprising (a) a substitution of one or more amino acid residues with other amino acid residues and/or (b) a deletion of one or more amino acid residues, wherein the substitution or deletion is carried out at a position in the region of: 217INVKRGKRYR226, 303LDPTNVFAVL312, 356VSLNLAIGRSTVDGIL371, 416VIELNITGGADHPI429, or 474GPWFVHCHIDWHLEAGLALVF494 of *Rhizoctonia solani* laccase.

The present invention also relates to mutants of *Myceliophthora* laccase, comprising (a) a substitution of one or more amino acid residues with other amino acid residues, (b) an insertion of one or more amino acid residues and/or (c) a deletion of one or more amino acid residues, wherein the substitution, insertion or deletion is carried out at a position corresponding to: 222GRRHRLRLIN231, 308AIFHYAGAPG317, 361VTLDTTGTPLFVWKVN376, 421ENDPGAPFTLPHPM433, or 497GAWLFHCHIAWHVSGGLGV515 of *Myceliophthora thermophila* laccase.

Preferably, each mutation is a substitution of one or more amino acid residues with other amino acid residues.

In a preferred embodiment, the mutants comprise a mutation in the segment corresponding to 416VIELNITGGADHPI429 of *Rhizoctonia solani* laccase and 421ENDPGAPFTLPHPM433 of *Myceliophthora thermophila* laccase.

The mutants of the present invention have a different pH-activity profile than the wild-type laccases, e.g., the mutants have a higher or lower pH optimum by an alteration of the charge distribution (or dielectric anisotrophy) at the T1 copper site. In order to enhance the activity of the laccase of interest in a more alkaline pH range, electron density and/or negative charge should be increased. Thus, in the mutants of the present invention, (a) a neutral amino acid residue is substituted with a negative amino acid residue or (b) a positive amino acid residue is substituted with a negative or neutral amino acid residue. In addition, neutral residues equipped with a functional group that bear a relatively high electron density and could act as general base, such as histidine, serine, threonine, tyrosine, cysteine, and methionine, may also be used to substitute other neutral residues possessing only simple aliphatic or aromatic side chains, such as leucine and phenylalanine. In order to enhance the activity of the laccase of interest in a more acidic pH range, electron density and/or negative charge should be decreased. Thus, in this embodiment of the mutants of the present invention, (a) a neutral amino acid residue is substituted with a positive amino acid residue or (b) a negative amino acid residue is substituted with a positive or neutral amino acid residue.

The Type 1 (T1) copper site consists of four ligands which bind to a copper ion, each of which is either an amino acid residue of the laccase or a small molecule such as a water molecule. A ligand is defined herein an amino acid residue of a laccase which binds to a copper ion. The Type 1 copper site of all known blue copper oxidases consists of the following ligands: two histidines (H), one cysteine (C), and, possibly, one additional methionine.

The ligand location for *Rhizoctonia solani* is: H 427, C 480, H 485 and possibly L 470 and for *Myceliophthora thermophilum* is H 431, C 503, H 508 and possibly L 513.

The present invention also relates to a method of altering the pH activity profile of a fungal laccase, comprising introducing a mutation selected from the group consisting of (a) a substitution of one or more amino acid residues with other amino acid residues, (b) an insertion of one or more amino acid residues and/or (c) a deletion of one or more amino acid residues, wherein the mutation is introduced in a segment corresponding to: 217INVKRGKRYR226, 303LDPTNVFAVL312, 356VSLNLAIGRSTVDGIL371, 416VIELNITGGADHPI429, 474GPWFVHCHIDWHLEAGLALVF494 of *Rhizoctonia solani* laccase, and 222GRRHRLRLIN231, 308AIFHYAGAPG317, 361VTLDTTGTPLFVWKVN376, 421ENDPGAPFTLPHPM433, 497GAWLFHCHIAWHVSGGLGV515 of *Myceliophthora thermophila* laccase.

In a preferred embodiment, the fungal laccase is a *Rhizoctonia* laccase (preferably a *Rhizoctonia solani* laccase or RsL; WO 95/07988) or a *Myceliophthora* laccase (preferably a *Myceliophthora thermophilum* laccase or MtL described in U.S. patent No. 5,795,760).

In another preferred embodiment, the fungal laccase is another *Rhizoctonia* laccase (as disclosed in U.S. patent No. 5,480,801), another *Myceliophthora* laccase (as disclosed in U.S. patent No. 5,795,760), and laccases of *Polyporus* (as disclosed in U.S. patent No. 5,770,418), *Trametes, Pyricularia, Coriolus, Scytalidium* (as disclosed in U.S. patent No. 5,843,745), *Rigidoporus* and *Phenllinus* (Geiger *et al.,* 1986, *Appl. Biochem. Biotech.,* 13: 97-110), *Podospora* (Moltitoris and Reinhammar, 1974, *Biochimica Biophysica Acta* 386: 493-502), *Lentinus* (Leatham and Stahmann, 1980, *Journal of General Microbiology* 125: 147-157), *Neurospora* (Germann *et al.,* 1987, *Journal of Biological Chemistry* 263: 885-896), *Aspergillus* (Kurtz and Champe, 1982, *Journal of Bacteriology* 151: 1338-1345), *Phlebia* (Niku-Paavola *et al.,* 1988, *Biochemical Journal* 254:877-884), *Botrytis* (Dubernet *et al.,* 1976, *Phytochemistry* 16: 191-193,), *Sclerotia* (Chet and Huttermann, 1982, *FEMS Microbiological Letters* 14: 211-215), *Curvularia* (Banerjee and Vohra, 1991, *Folia Microbiol.* 36: 343-346), *Fomes* (Haars and Huttermann, 1983, *Arch. Microbiol.* 134: 309-*313), Schizophyllum* (De Vries *et al.,* 1986, *Journal of General Microbiology* 132: 2817-2826), *Cerrena* (Bekker *et al.,* 1990, *Biokhimia* 55: 2019-2024), *Armillaria* (Rehman and Thurston, 1992, *Journal of General Microbiology* 138: 1251-1257), *Agaricus* (Perry *et al.,* 1993, *Journal of General Microbiology* 139: 1209-1218), *Pleurotus* (Von Hunolstein *et al.,* 1986, *Journal of General Applied Microbiology* 32: 185-191), *Acer pseudopaltanus* (Lafayette *et al.,* 1995, *Plant Physiology* (Rockville) 107: 667-668), and *Rhus* (Bertrand, 1895, C. *R. Acad. Sci.* Paris 121: 166).

The mutants of the present invention may have a different specific activity than the wild-type laccases. For example, a negative charge, or more precisely, a relatively high electron density, in the T1 copper site region is important for activity.

The present invention also relates to mutants which can be expressed in higher yields. Such mutants include laccases comprising a substitution of a phenylalanine with another amino acid residue. For example, substituting phenylalanine at a position corresponding to residue 513 of *Myceliophthora thermophila* laccase and position 470 in *Rhizoctonia solani* isozyme 4 laccase results in a low expression yield. Thus, the mutants of the present invention encompass substitutions of Phe at one of these positions with another amino acid residue. Preferably, the amino acid residue does not ligate to copper, i.e., the amino acid residue is not histidine, cysteine, methionine, glutamate, and aspartate. Preferably, phenylalanine is substituted by leucine. In a preferred embodiment, the yield of the mutant enzyme is increased at least two-fold, more preferably at least five-fold, over the yield observed with the corresponding wild-type enzyme when both are expressed in the same host and fermented under the same conditions.

In a preferred embodiment, the mutants comprise at least two amino acid residues, more preferably at least 3 amino acid residues. In another preferred embodiment, the mutants comprise five mutations, more preferably four mutations, even more preferably three mutations, even more preferably two mutations, and most preferably one mutation.

The mutants described herein are most efficiently prepared by site-directed mutagenesis of the DNA encoding the wild-type laccase of interest. Such techniques are well-known in the art, and are described in, for example, Sambrook *et al.*, 1989, *Molecular Cloning, A Laboratory Manual,* Cold Spring Harbor Laboratory, Cold Spring Harbor, New York. The present invention also encompasses the nucleic acid encoding the mutant laccases, as well as vectors and host cells comprising same, for use in recombinant expression of the mutant enzyme.

The choice of host cells and expression vectors will to a large extent depend upon the enzyme of choice and its source. The mutant gene can be expressed, in active form, using an expression vector. A useful expression vector contains an element that permits stable integration of the vector into the host cell genome or autonomous replication of the vector in a host cell independent of the genome of the host cell, and preferably one or more phenotypic markers which permit easy selection of transformed host cells. The expression vector may also include control sequences encoding a promoter, ribosome binding site, translation initiation signal, and, optionally, a repressor gene, a selectable marker or various activator genes. To permit the secretion of the expressed protein, nucleotides encoding a signal sequence may be inserted prior to the coding sequence of the gene. For expression under the direction of control sequences, a laccase gene to be used according to the invention is operably linked to the control sequences in the proper reading frame. Promoter sequences that can be incorporated into plasmid vectors, and which can direct the transcription of the laccase gene, include, but are not limited to, the prokaryotic β-lactamase promoter (Villa-Kamaroff *et al.* ,1978, *Proceedings of the National Academy of Sciences USA* 75: 3727-3731) and the *tac* promoter (DeBoer *et al. ,* 1983, *Proceedings of the National Academy of Sciences USA* 80: 21-25). Further references can also be found in "Useful proteins from recombinant bacteria" in 1980, *Scientific American* 242: 74-94; and in Sambrook *et al. ,* 1989, *supra.*

The expression vector carrying the nucleic acid construct of the invention may be any vector which may be conveniently subjected to recombinant DNA procedures. The vector may be an autonomously replicating vector, *i.e.* , a vector which exists as an extrachromosomal entity, the replication of which is independent of chromosomal replication, *e.g.,* a plasmid, or an extrachromosomal element, minichromosome or an artificial chromosome. Alternatively, the vector may be one which, when introduced into a host cell, is integrated into the host cell genome and replicated together with the chromosome(s) into which it has been integrated.

In the vector, the nucleic acid sequence should be operably connected to a suitable promoter sequence. The promoter may be any DNA sequence which shows transcriptional activity in the host cell of choice and may be derived from genes encoding proteins either homologous or heterologous to the host cell. Examples of suitable promoters for directing the transcription of the DNA construct of the invention, especially in a bacterial host, are the promoter of the lac operon of *E. coli,* the *Streptomyces coelicolor* agarase gene *dagA* promoters, the promoters of the *Bacillus licheniformis* alpha-amylase gene *(amyL),* the promoters of the *Bacillus stearothermophilus* maltogenic amylase gene *(amyM).* the promoters of the *Bacillus amyloliquefaciens* alpha-amylase *(amyQ),* or the promoters of the *Bacillus subtilis xylA* and *xylB* genes. In a yeast host, a useful promoter is the ENO-1 promoter. For transcription in a fungal host, examples of useful promoters are those derived from the gene encoding *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase *(glaA), Rhizomucor miehei* lipase, *Aspergillus oryzae* alkaline protease, *Aspergillus oryzae* triose phosphate isomerase or *Aspergillus nidulans* acetamidase. Preferred are the TAKA-amylase and *glaA* promoters.

The expression vector of the invention may also comprise a suitable transcription terminator and, in eukaryotes, polyadenylation sequences operably connected to the DNA sequence encoding the laccase of the invention. Termination and polyadenylation sequences may suitably be derived from the same sources as the promoter. The vector may further comprise a DNA sequence enabling the vector to replicate in the host cell in question. Examples of such sequences are the origins of replication of plasmids pUC19, pACYC177, PUB110, pE194, pAMB1 and pIJ702.

The vector may also comprise a selectable marker, e.g. , a gene the product of which complements a defect in the host cell, such as the dal genes from *Bacillus subtilis* or *Bacillus licheniformis,* or one which confers antibiotic resistance such as ampicillin, kanamycin, chloramphenicol or tetracycline resistance. Examples of *Aspergillus* selection markers include *amdS, pyrG, argB, niaD, sC,* and *hygB* a marker giving rise to hygromycin resistance. Preferred for use in an *Aspergillus* host cell are the *amdS* and *pyrG* markers of *Aspergillus nidulans* or *Aspergillus oryzae.* A frequently used mammalian marker is the dihydrofolate reductase (DHFR) gene. Furthermore, selection may be accomplished by co-transformation, *e.g.*, as described in WO 91/17243.

It is generally preferred that expression gives rise to a product that is extracellular. The laccases of the present invention may thus comprise a preregion permitting secretion of the expressed protein into the culture medium. If desirable, this preregion may be native to the laccase of the invention or substituted with a different preregion or signal sequence, conveniently accomplished by substitution of the DNA sequences encoding the respective preregions. For example, the preregion may be derived from a glucoamylase or an amylase gene from an *Aspergillus* species, an amylase gene from a *Bacillus* species, a lipase or proteinase gene from *Rhizomucor miehei,* the gene for the alpha-factor from Saccharomyces cerevisiae, or the calf preprochymosin gene. Particularly preferred, when the host is a fungal cell, is the preregion for *Aspergillus oryzae* TAKA amylase, *Aspergillus niger* neutral amylase, the maltogenic amylase form *Bacillus* NCIB 11837, *Bacillus stearothermophilus* alpha-amylase, or Bacillus licheniformis subtilisin. An effective signal sequence is the *Aspergillus oryzae* TAKA amylase signal, the *Rhizomucor miehei* aspartic proteinase signal, and the *Rhizomucor miehei* lipase signal.

The procedures used to ligate the nucleic acid construct of the invention, the promoter, terminator and other elements, respectively, and to insert them into suitable vectors containing the information necessary for replication, are well known to persons skilled in the art *(cf.* for instance, Sambrook *et al.,* 1989, *supra).*

The cell of the invention either comprising a nucleic acid construct or an expression vector of the invention as defined above is advantageously used as a host cell in the recombinant production of an enzyme of the invention. The cell may be transformed with the nucleic acid construct of the invention, conveniently by integrating the construct into the host chromosome. This integration is generally considered to be an advantage as the sequence is more likely to be stably maintained in the cell. Integration of the constructs into the host chromosome occurs by homologous or heterologous recombination. Alternatively, the cell may be transformed with an expression vector as described above in connection with the different types of host cells.

The host cell may be selected from prokaryotic cells, such as bacterial cells. Examples of suitable bacteria are gram positive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis,* or *Streptomyces lividans* or *Streptomyces murinus,* or gram negative bacteria such as *E. coli.* The transformation of the bacteria may, for instance, be effected by protoplast transformation or by using competent cells in a manner known *per se.*

The host cell is preferably a eukaryote, such as mammalian cells, insect cells, plant cells or preferably fungal cells, including yeast and filamentous fungi. For example, useful mammalian cells include CHO or COS cells. A yeast host cell may be selected from a species of *Saccharomyces* or *Schizosaccharomyces, e. g. , Saccharomyces cerevisiae.* Useful filamentous fungi may be selected from a species of *Aspergillus, e.g., Aspergillus oryzae or Aspergillus niger.* Alternatively, a strain of a *Fusarium* species, *e.g. , Fusarium oxysporum,* or *Fusarium graminearum,* can be used as a host cell. Fungal cells may be transformed by a process involving protoplast formation and transformation of the protoplasts followed by regeneration of the cell wall in a manner known *per se.* A suitable procedure for transformation of *Aspergillus* host cells is described in EP 238 023. A suitable method of transforming *Fusarium* species is described by Malardier *et al.* , 1989, *Gene* 78:147-156 or in copending U.S. application Serial No. 08/269,449.

The present invention thus also provides a method of producing a recombinant protein of the invention, which method comprises cultivating a host cell as described above under conditions conducive to the production of the enzyme and recovering the enzyme from the cells and/or culture medium. The medium used to cultivate the cells may be any conventional medium suitable for growing the host cell in question and obtaining expression of the laccase of the invention. Suitable media are available from commercial suppliers or may be prepared according to published formulae (*e.g.*, in catalogues of the American Type Culture Collection).

The resulting enzyme may be recovered from the medium by conventional procedures including separating the cells from the medium by centrifugation or filtration, precipitating the proteinaceous components of the supernatant or filtrate by means of a salt, *e.g.,* ammonium sulphate, followed by purification by a variety of chromatographic procedures, *e.g.,* ion exchange chromatography, gel filtration chromatography, affinity chromatography, or the like. Preferably, the isolated protein is about 90% pure as determined by SDS-PAGE, purity being most important in food, juice or detergent applications.

In a particularly preferred embodiment, the expression of the enzyme is achieved in a fungal host cell, such as *Aspergillus.* As described in detail in the following examples, the laccase gene is ligated into a plasmid containing the *Aspergillus oryzae* TAKA alpha-amylase promoter, and the *Aspergillus nidulans amdS* selectable marker. Alternatively, the *amdS* may be on a separate plasmid and used in co-transformation. The plasmid (or plasmids) is used to transform an *Aspergillus* species host cell, such as *Aspergillus oryzae* or *Aspergillus niger* in accordance with methods described in Yelton *et al. ,* 1984, *Proceedings of the National Academy of Sciences USA* 81: 1470-1474.

The modified oxidases, particularly laccases of the present invention can be used in a number of industrial methods. These processes include polymerization of lignin, both Kraft and lignosulfates, in solution, in order to produce a lignin with a higher molecular weight. Such methods are described in, for example, Jin *et al.,* 1991, *Holzforschung* 45: 467-468; U.S. Patent No. 4,432,921; EP 0 275 544; PCT/DK93/00217, 1992.

The oxidases of the present invention can also be used for in situ depolymerization of lignin in Kraft pulp, thereby producing a pulp with lower lignin content. This use of these enzymes is an improvement over the current use of chlorine for depolymerization of lignin, which leads to the production of chlorinated aromatic compounds, which are an environmentally undesirable by-product of paper mills. Such uses are described in, for example, *Current Opinion in Biotechnology* 3: 261-266, 1992; *Journal of Biotechnology 25:* 333-339, 1992; Hiroi *et al.*, 1976, *Svensk Papperstidning 5:* 162-166.

Oxidation of dyes or dye precursors and other chromophoric compounds leads to decolorization of the compounds. Laccase can be used for this purpose, which can be particularly advantageous in a situation in which a dye transfer between fabrics is undesirable, e.g., in the textile industry and in the detergent industry. Methods for dye transfer inhibition and dye oxidation can be found in WO 92/01406, WO 92/18683, EP 0495836 and Calvo, *1991, Mededelingen van de Faculteit Landbouw-wetenschappen*/*Rijiksuniversitet Gent.* 56: 1565-1567; Tsujino *et al.*, 1991, *Journal of the Chemical Society* 42: 273-282; methods for the use of oxidation of dye and dye precursors in hair coloring are found in U.S. patent No. 5,795,760 and 5,770,418.

The present laccase can also be used for the polymerization of phenolic or aniline compounds present in liquids. An example of such utility is the treatment of juices, such as apple juice, so that the laccase will accelerate a precipitation of the phenolic compounds present in the juice, thereby producing a more stable juice. Such applications have been described in Stutz, 1993, *Fruit Processing* 7/93, 248-252; Maier *et al.,* 1990, *Dt. Lebensmittel-rindschau* 86: 137-142; Dietrich *et al.,* 1990, *Fluss. Obst* 57: 67-73.

The present invention is further explained in the following non-limiting examples.

### Examples

### Materials and methods

Chemicals used as buffers and substrates are commercial products of at least reagent grade.

The protocols for molecular biology experiments (including restriction digests, DNA ligations, gel electrophoresis, and DNA preparations) are adapted from either the instructions of the manufacturer or standard procedures (Sambrook *et al.,* 1989, supra). All oligonucleotides are synthesized by an Applied Biosystems 294 DNA/RNA Synthesizer. Nucleotide sequences are determined by an Applied Biosystems automatic DNA Sequencer, Model 373A, version 1.2.0.

### Example 1: Site-directed mutagenesis of Myceliophthora thermophila laccase

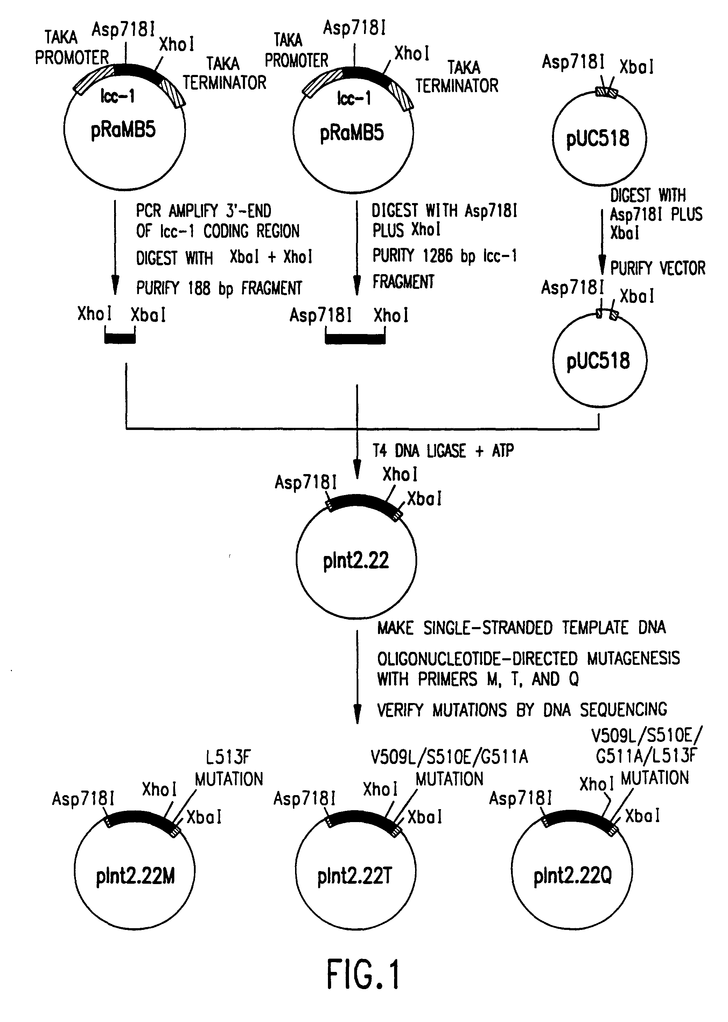

The construction of a *Myceliophthora thermophila* laccase expression vector, pRaMB17, and several derivatives, pRaMB 17M, pBANe22T, and pRaMB17Q, which direct expression of the *Myceliophthora thermophila* wild-type laccase and laccase variants, is shown in Figures 1-4. The primers used in the constructions are summarized in Table 1.

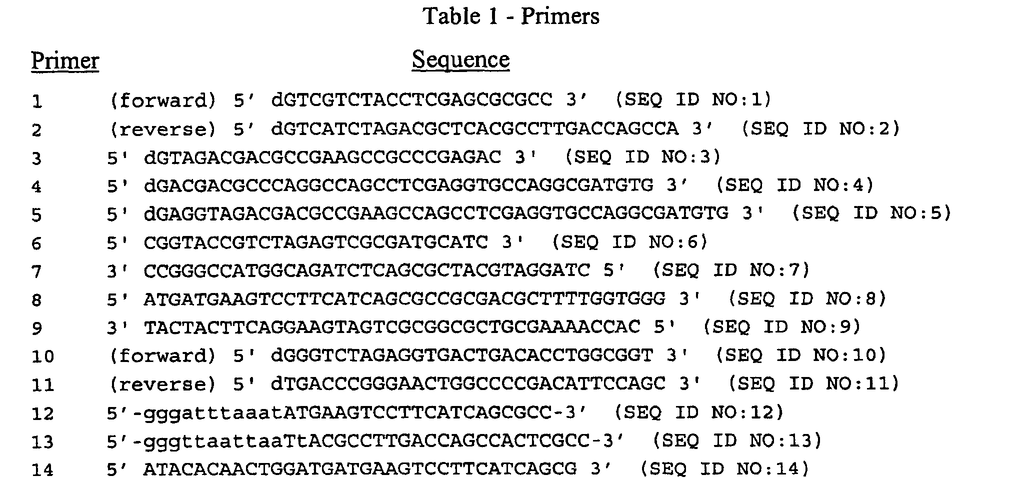

Specifically, a small DNA fragment containing the 3'-terminus of the *lcc-1* coding region (including stop codon) is generated by PCR using pRaMB5 (U.S. patent No. 5,795,760) as a template for *Pfu* polymerase with primers 1 and 2 listed in Table 1. The 188 bp PCR product is digested with *Xba*I plus *Xho*I and purified by agarose gel electrophoresis. The purified fragment is then mixed in a three-part ligation reaction with an *Asp*718I-*Xho*I segment (1286 bp) of the *lcc-1* gene from pRaMB5, and pUC518 (a derivative of pUC118; Vieira and Messing, 1987, *Methods in Enzymology* 153: 3-4), containing additional restriction sites for *Bgl*II, *Cla*I, *Xho*I and *Nsi*I in the polylinker, which has been cleaved with *Asp*718I-*Xba*I. The resulting plasmid, pInt2.22, which contains approximately 1.5 kb of the *lcc-1* coding region, is extended from an internal *Asp*718I site through the stop codon which is followed immediately by a *Xba*I site. Single-stranded pInt2.22 DNA template is prepared (Vieira and Messing, 1987, *supra)* and used as a template for oligonucleotide-directed mutagenesis *(Adelman et al.,* 1983, *DNA* 2: 183-193) with primer 3 for L513F mutation, primer 4 for V509L/S510E/G511A mutation, and primer 5 for V509L/S510E/G511A/L513F mutation to derive the precursor plasmids for pRaMB17, pRaMB17M, pBANe22T and pRaMB17Q.

Mutants are identified by hybridization with radiolabeled oligonucleotide primers 3, 4, and 5, and each mutation is verified by DNA sequence analysis.

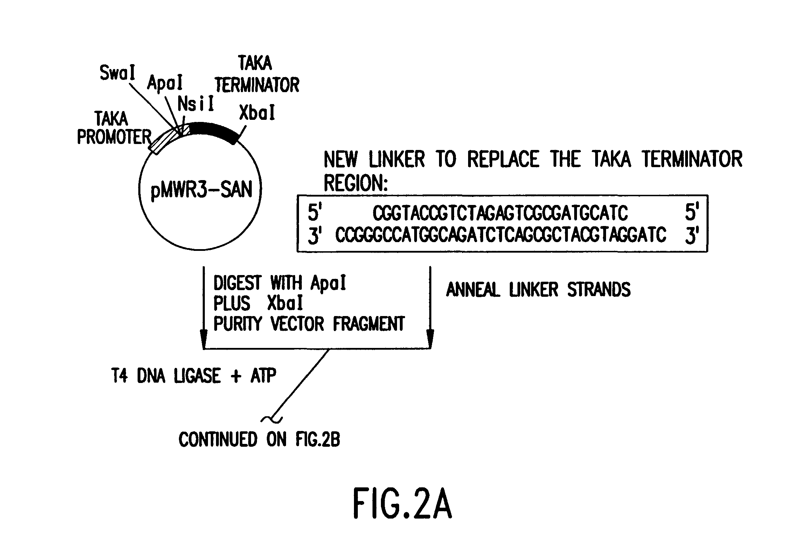

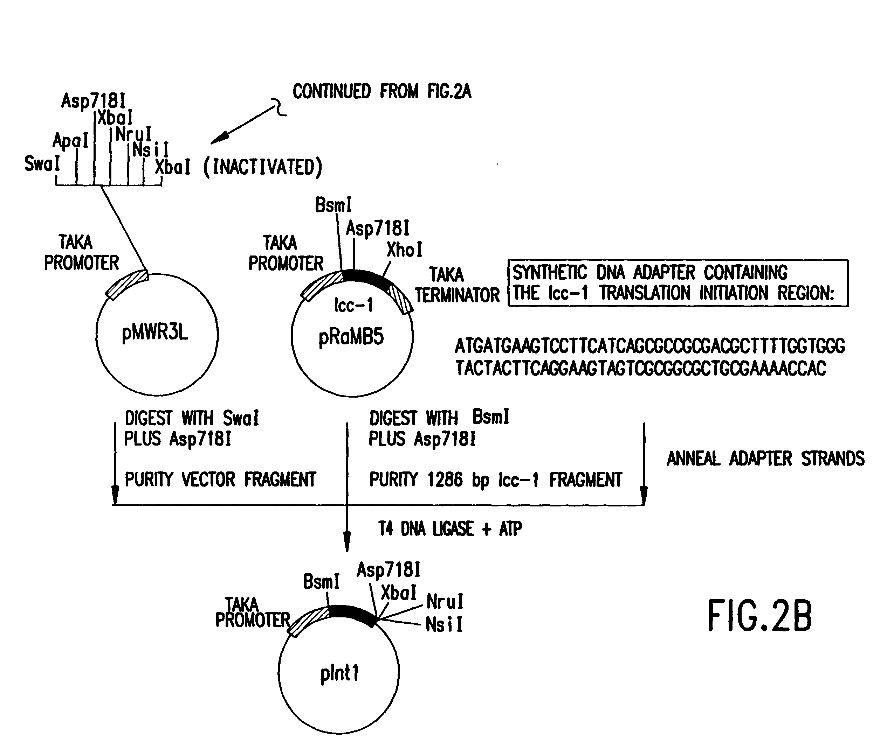

The next step in the construction of pRaMB17 and its derivatives is partially shown in Figure 2. The starting plasmid, pMWR3-SAN, is prepared by cleaving bacteriophage vector M13mp18 *(Yanisch-Perron et al.*, 1985, *Gene* 33: 103-119) with *HindIII* and *EcoRI,* and purifying the large vector fragment by agarose gel electrophoresis. This fragment is ligated with a synthetic DNA linker having the following sequence:

5'AATTCGTCGACGGYCTCTATTTCTGTACGGCCTTCAGGTGGCCGCACCGGCCA
TGCATAGCAGCTGCCAGAGATAAAGACATGCCGGAAGTCCACCGGCGTGGCCG
GTACGTATTCGA 3' (SEQ ID NO:15)

The resulting phage vector, mp18-5'link, is then digested with *Sal*I and *Bsa*I (both sites in the synthetic linker region) and ligated with a 1.1 kb *Sal*I-*Bsa*I fragment from pTAKA-17 comprising the TAKA promoter region to generate the recombinant phage mp18-5'. Plasmid pUC18 (Yanisch-Perron *et al., 1985,* supra) is digested with *Hind*III plus *Eco*RI and the 2.6 kb vector fragment is purified by agarose gel electrophoresis. The isolated fragment is ligated with a synthetic linker with the following sequence:

5' AATTGTTTAAACTCTAGAGAATTCAAGCTTGTCGACGTTTAAACCAAATTT
GAGATCTCTTAAGTTCGAACAGCTGCAAATTTGTCGA 3' (SEQ ID NO:16)

The resulting plasmid, pUC18::TAKA-link, is digested with *Sal*I plus *Eco*RI and the vector fragment is isolated by agarose gel electrophoresis. pTAKA-17 is used as a template for PCR amplification of a 0.7 kb TAKA-amylase terminator fragment. For this purpose, the following primers are used:
forward primer: 5' dATGCATAGGGTGGAGAGTATATGATGG 3' (SEQ ID NO:17) reverse primer:

5' dCTGAATTCCGTTTCGTTTAC 3' (SEQ ID NO:18)

The 0.7 kb product of this PCR reaction is digested with *NsiI* plus *EcoRI* and mixed in a three-part ligation with *Sal*I and *Eco*RI cleaved pUC18::TAKA-link and the 1.1 kb SaII-NsiI TAKA promoter fragment from mp18-5' to produce pMWR1.

Plasmid pMWRI is modified to generate pMWR3. First, a new TAKA-amylase promoter segment is generated by PCR using pTAKA-17 as a template with the following synthetic primers:
forward primer: 5' dTCCTGCAGAATGCAATTTAAACTC 3' (SEQ ID NO:19) reverse primer:

5' dCTATGCATATTTAAATGCCTTCTGTGGGGTTTATTG 3' (SEQ ID NO:20)

The 0.2 kb PCR product is digested with *Nsi*I plus *Pst*I and ligated with the large vector fragment of pMWR1 which has been cleaved with *Nsi*I and *Pst*I. The resulting plasmid, pMWR3, is then modified by inserting a small linker, AATTGGGCCCATGCA (SEQ ID NO:21), which contains an *Apa*I site between the *Swa*I and *Nsi*I sites, creating pMWR3-SAN. A derivative of pMWR3-SAN is then constructed by replacing the *Apa*I-*Xba*I TAKA-amylase terminator fragment with a small linker (primers 6 and 7 shown in Table 1). This linker introduces *Asp*718I, *Xba*I, and *Nru*I cloning sites and inactivates the *Xba*I site of pMWR3-SAN yielding pMWR3L.

pMWR3L is digested with *Swa*I and *Asp*718I and mixed in a three-part ligation with a 853 bp *Bsm*I-*Asp*718I fragment comprising the 5'-end of the *lcc-1* coding region and synthetic DNA adapter containing the translation initiation region (primers 8 and 9 shown in Table 1) to yield plasmid pInt1.

A 597 bp DNA segment comprising the *Aspergillus niger glaA* terminator region is then isolated by PCR using pHD414 (EP 238 023) as a template with primers 10 and 11 shown in Table 1, which introduce *Xba*I and *Sma*I sites at the 5' and 3'-ends of the terminator, respectively. The amplified DNA fragment is subsequently cleaved with *Xba*I plus *Sma*I and subcloned into pUC118 to generate plasmid pUC::AMGterm.

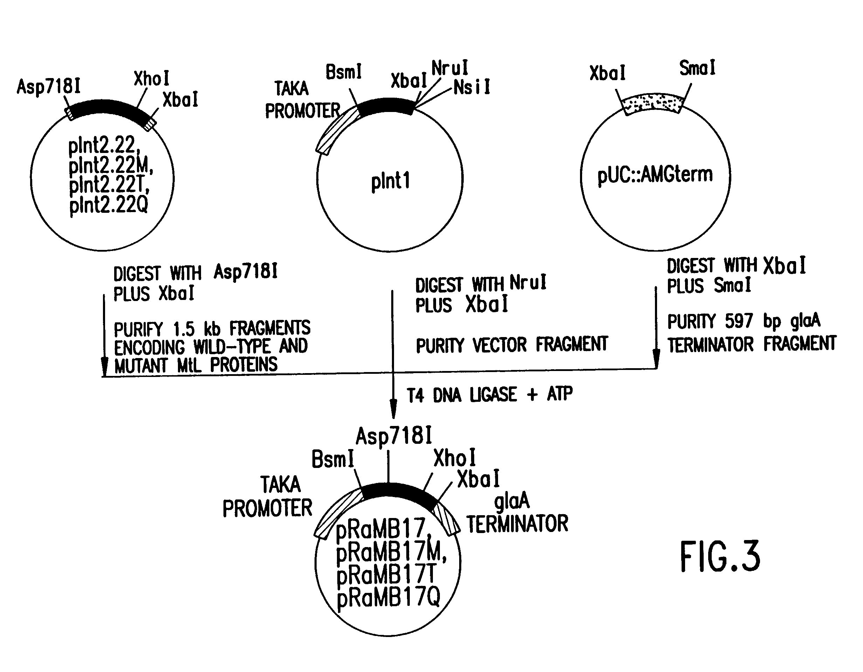

Finally, the 1.5 kb fragments containing the wild-type and mutant *lcc-1* gene sequences are excised by digestion with *Asp*718I and *Xba*I and purified by agarose gel electrophoresis. Each of these fragments is mixed in a three part ligation (Figure 3) with *Asp*718I and *Nru*I digested pInt1 plus the 597 bp *Xba*I-*Sma*I *glaA* terminator fragment from pUC::AMGterm to produce pRaMB17, pRaMB17M, pRaMB17T and pRaMB17Q.

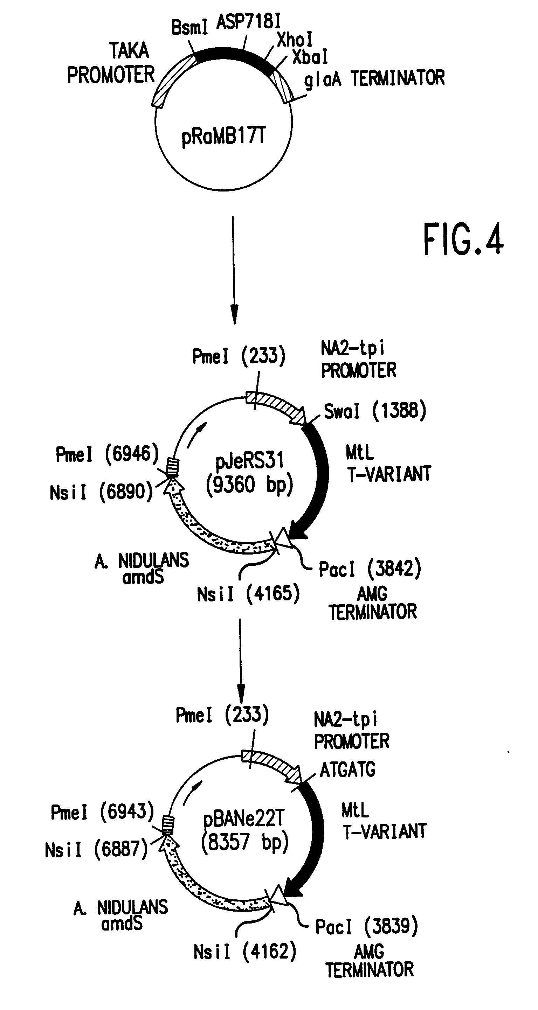

DNA primers 12 and 13 (uppercase letters represent sequences in the laccase gene) are used in a PCR reaction to amplify the mutant laccase gene from plasmid pRaMB17T (Figure 4). The PCR is performed in a 50 ml reaction containing 120 ng of plasmid pRaMB17T, 0.05 mM each of dATP, dTTP, dGTP, dCTP, 100 pmol each of primers 12 and 13, 1X *Pwo*I Buffer (Boehringer Mannheim, Indianapolis, IN), 5% (v/v) DMSO, and 2.5 units *Pwo*I (Boeringer Mannheim, Indianapolis, IN). The PCR conditions are 95°C for 3 minutes, 30 cycles each at 95°C for 1 minute, 60°C for 1 minute, and 72°C for 1.5 minutes, and then 72°C for 5 minutes. The PCR reaction mixture is run on a agarose gel and the 2.4 kb DNA laccase band is excised. The DNA is purified by solubilization of the agarose with 3 volumes Qia-ex solubilization buffer (Qiagen, Los Angeles, CA) followed by a Qiaquick PCR spin column according to the manufacturer's directions (Qiagen, Los Angeles, CA). The DNA is recovered in 50 ml of 1 mM EDTA-10 mM Tris pH 8 buffer. A 20 µl aliquot of the DNA is cut in a final volume of 25 µl containing 1X restriction enzyme buffers and restriction enzymes *Pac*I and *Swa*I as suggested by the manufacturer. The reaction mixture is then heated at 80°C for 10 minutes. One ml of the *Pac*I/*Swa*I cut laccase gene is ligated into *Pac*I/*Swa*I cut plasmid pBANe6. The ligation mixture is used to transform *E. coli* strain DH5α. The plasmid containing pBANe6 and the mutant laccase sequences is designated pJeRS31. pJeRS31 is subjected to site-directed mutagenesis using primer 14 to remove the *Swa*I site and add a second ATG using the MORPH Site-Specific Plasmid DNA Mutagenesis Kit according to the manufacturer's instructions (5 Prime 3 Prime, Inc., Boulder, CO) to produce pBANe22T.

A summary of the plasmids is provided in Table 2.

**Table 2.**

| pRaMB17 and its derivatives | |
|---|---|
| Vector | MtL protein encoded |
| pRaMB17 | Wild-type MtL |
| pRaMB17M | MtL with the L513F mutation |
| pBANe22T | MtL with the triple substitution V509L/S510E/G511A |
| pRaMB17Q | MtL with the quadruple substitution V509L/S510E/G511A/L513F |

### Example 2: Transformation of Aspergillus oryzae with modified Myceliophthora thermophila laccase genes

Methods for co-transformation of *Aspergillus oryzae* are described by Christensen *et al.,* 1988, *Bio*/*Technology* 6: 1419-1422. For introduction of each of the *Myceliophthora thermophila* laccase expression vectors pRamB17, pRamB17M, pBANe22T, and pRaMB17Q into *Aspergillus oryzae* HowB711, equal amounts (approximately 5 µg each) of the laccase expression vector and pToC90 (WO 91/17243) are added to approximately 106 protoplasts in suspension while pBANe22T is added alone. Transformants are selected on Cove medium (Cove, 1966, *Biochimica Biophysica Acta* 113: 51-56) containing 1 M sucrose, 10 mM acetamide as the sole nitrogen source, and 20 mM CsCl to inhibit background growth. The transformants selected in this way are subsequently screened for the ability to produce laccase on Cove medium containing 1-3 mM ABTS. Cells which secrete active laccase oxidize the ABTS, producing a green halo surrounding the colony. Transformants which produce detectable laccase activity on ABTS plates are purified twice through conidiospores.

### Example 3: Expression of modified Myceliophthora thermophila laccases

The transformants described in Example 2 are grown in shake flask cultures containing 25 ml of ASPO4 medium (pRaMB17, pRaMB17M, pRaMB17Q) or MY51 medium (pBANe22T) for 4 to 5 days at 37°C. ASPO4 medium is comprised of 1 g of CaCl₂-2H₂O, 2 g of yeast extract, 1 g of MgSO₄, 2 g of citric acid, 5 g of KH₂PO₄, 1 g of urea, 2 g of (NH₄)₂SO₄, 20 g of maltodextrin, and 0.5 ml of trace metals solution per liter. MY51 medium is comprised of 50 g of maltodextrin, 2 g MgSO₄-7H₂0, 10 g of KH₂PO₄, 2 g of citric acid, 10 g of yeast extract, 2 g of urea, 1 g of urea, 2 g of (NH₄)₂SO₄, and 0.5 ml of trace metals solution. The trace metals solution is comprised of 14.3 g of ZnSO₄-7H₂0, 2.5 g of CuSO₄-5H₂O, 0.5 g of NiCl₂-6H₂O, 13.8 g of FeSO₄-7H₂0, 8.5 g of MgSO₄-H₂O, and 3.0 g of citric acid per liter of RO water. Culture supernatants are assayed for laccase activity using either ABTS or syringaldazine as a substrate as described below.

Syringaldazine (SGZ) oxidation is determined in MES pH 5.3 buffer or Britten-Robinson buffer, pH 2.7 to 11.0, with 10 % ethanol (coming from SGZ stock solution) by monitoring the absorbance change at 530 nm with an extinction coefficient of 65 mM⁻¹cm⁻¹ (Bauer and Rupe, 1971, *Analytical Chemistry* 43: 421-425) at 20°C. Laccase activity using SGZ as a substrate is assayed by mixing 800 µl of assay buffer (40 µM CuSO₄-25 mM sodium acetate pH 5.5) with 20 µl of culture supernatant and 60 µl of 0.28 mM syringaldazine in 50% ethanol. The absorbance at 530 nm is measured over time in a UV-VIS spectrophotometer. One laccase unit (LACU) is defined as the amount of enzyme which oxidizes one µmole of substrate per minute at 30°C.

ABTS oxidation is determined at pH 5 in a 96-well plate at 20°C by monitoring the absorbance change at 405 nm with an extinction coefficient of 35 mM⁻¹cm⁻¹ (Childs and Bardsley, 1975, *Biochemical Journal* 145: 93-103). Laccase activity using ABTS as a substrate is measured by mixing 20 µl of culture supernatant with 200 µl of substrate solution containing 0.275 mg of ABTS per ml of 100 mM sodium acetate pH 5.0.

Shake flask cultures producing high levels of extracellular laccase activity are further evaluated by fermentation. A 1 ml aliquot of a spore suspension (approximately 109 spores) of an *Aspergillus oryzae* transformant expressing the laccase variant of interest is added aseptically to each of several 500 ml shake flasks containing 100 ml of medium comprised of 50 g of Nutriose 725, 2 g of MgSO₄-7H₂O, 10 g of KH₂PO₄, 2 g of K₂SO₄, 0.5 g of CaCl₂-2H₂0, 2 g of citric acid, 10 g of yeast extract, 0.5 ml of trace metals (as described above), and 2 g of urea per liter of tap water (adjusted to pH 6.0 before autoclaving) and incubated at 34°C on a rotary shaker at 200 rpm for about 18 hours. Samples of the shake flask broths are then transferred to a laboratory fermentor containing medium, supplemented with 2 mM CuSO₄-5H₂O, comprised of 30 g of Nutriose, 5 g of yeast extract, 2 g of (NH₄)₂HPO₄, 2 g of MgSO₄-7H₂O, 4 g of citric acid, 3 g of K₂SO₄, 2 g of CaCl₂-H₂O, and 0.5 ml of trace metals solution (as described above) per liter and fed during the course of the fermentation with a medium comprised of 270 g of Nutriose, 30 g of urea, and 15 g of yeast extract per liter. The fermentaion is allowed to proceed at 31°C, pH 7, 600-700 rpm for 7 days.

Laccase yields for the "M" (L513F) and "T" (V509L/S510E/G511A) mutants from these fermentations are estimated to be 25% and 40%, respectively, of the wild-type yield. In contrast, the expression yield of mutant "Q" (V509L/S510E/G511A/L513F) is so low that there is insufficient laccase for purification.

### Example 4: Purification of modified Myceliophthora thermophila laccases

The wild-type, "M", and "T" fermentation broths from Example 3 are cheese-cloth filtered (pH 7.6, 16 mS), filtered through Whatman #2 filter paper, concentrated on a Spiral Concentrator (Amicon) with a S1Y100 membrane (100 kDa MW-CO), and diluted to 0.75 mS with glass distilled water. The washed concentrated broths are loaded onto a Q-Sepharose XK26 (Pharmacia, Uppsala, Sweden) column (120 ml), pre-equilibrated with 10 mM Tris, pH 7.5, 0.7 mS (Buffer A), and active fractions are eluted during the linear gradient with Buffer B (Buffer A plus 2 M NaCl). The active fractions are pooled, adjusted to 1 mS in ionic strength, and subjected to a Mono-Q (Pharmacia, Uppsala, Sweden) chromatography equilibrated with Buffer A. Laccase preparations with apparent electrophoretic purity are obtained in the run-through fractions.

### Example 5: Site-directed mutagenesis of Rhizoctonia solani laccase gene

Site-specific mutations are introduced into the *Rhizoctonia solani* laccase rs14 gene of the expression plasmid, pJiWa59, using the overlap-extension PCR method (Ho, 1989, *Gene* 77: 51-59) together with the primers listed in Table 3. Primer 15 (SEQ ID NO:22) is used to create pJiWa85 that encodes three amino acid changes ("T": L466V/E467S/A468G) in the laccase coding region (Table 3). PCR amplification with primer 16 (SEQ ID NO: 23) results in pJiWa86 which encodes a single amino acid mutation ("M": L470F). For each mutation, a 505 nt *Sac*I/*Nor*I fragment is generated by PCR and used to replace the homologous fragment in pJiWa59. PCR-amplified regions of the gene are sequenced to confirm the mutation as well as to ascertain the integrity of the coding region.

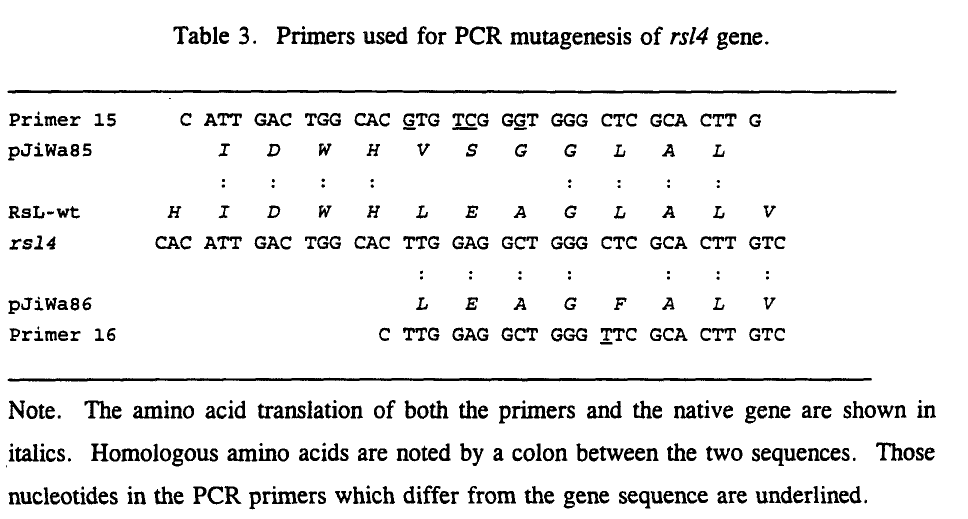

### Example 6: Transformation of Aspergillus oryzae with the modified Rhizoctonia solani rsl4 genes

*Aspergillus oryzae* HowB711 is transformed with 8 µg of pJiWa85 ("T": L466V/E467S/A468G) or pJiWa86 ("M": L470F) together with 2 µg of pToC90 and Aspergillus oryzae HowB104 is transformed with 8 µg of pJiWa59 (wt) together with 2 µg of pToC90 using a standard PEG mediated protocol (Yelton, 1984, *Proceedings of the National Academy of Sciences USA* 81: 1470-1474). The transformants are selected on Minimal medium plates supplemented with 10 mM acetamide and 1 M sucrose. The Minimal medium is comprised of 6.0 g of NaNO₃, 0.52 g of KCI, 1.52 g of KH₂PO₄, 1.0 ml of trace metals solution, 20 g of Nobel Agar (Difco), 20 ml of 50% glucose, 20 ml of methionine (50 g/l), 20 ml of biotin (200 mg/l), 2.5 ml of 20% MgSO₄-7H₂O, and 1.0 ml of mg/ml streptomycin per liter. The agar medium is adjusted to pH 6.5 prior to autoclaving and then glucose, methionine, biotin, MgSO₄-7H₂O, and streptomycin are added as sterile solutions to the cooled autoclaved medium and poured into plates. The trace metals solution is comprised of 0.04 g of Na₂B₄O₇-10H₂O, 0.4 g of CuSO₄-5H₂O, 1.2 g of FeSO₄-7H₂O, 0.7 g of MnSO₄-H₂O, 0.8 g of Na₂MoO₂-2H₂O, and 10 g of ZnSO₄-7H₂O per liter of RO water.

Laccase activity is scored on Minimal medium plates containing 10 mM acetamide and 1 g/l ABTS. Colonies that produce a green halo, indicative of laccase expression, are spore-purified twice.

### Example 7: Expression of modified Rhizoctonia solani laccases

The spores from transformants of pJiWa59 (wt), pJiWa85 ("T"), and pJiWa86 ("M") described in Example 6 are used to inoculate 15 ml of MY51 medium in 125 ml shake flasks. After 3 days and 5 days growth at 37°C, a 1 ml aliquot is removed from each shake flask and centrifuged at 14,000 g for 5 minutes to remove any mycelia clumps. The supernatants are assayed for ABTS oxidation in 96-well microtiter plates as described below.

ABTS oxidation is determined in MES pH 5.3 buffer or Britten-Robinson buffer at pH 2.7 to 11.0 in a 96-well plate at 20°C by monitoring the absorbance change at 405 nm with an extinction coefficient of 35 mM⁻¹cm⁻¹ (Childs and Bardsley, 1975, *Biochemical Journal* 145: 93-103).

The transformants yielding the highest laccase activity are selected for fermentation and grown as described in Example 3. Laccase yields for the "M" (L470F) and "T" (L466V/E467S/A468G) mutants from these fermentations are estimated to be 17 % and 50 % , respectively, of the wild-type yield.

### Example 8: Purification of Rhizoctonia solani modified laccases

The wild-type, "M", and "T" fermentation broths from Example 7 are cheese-cloth filtered (pH 7.6, 16 mS), filtered through Whatman #2 filter paper, and concentrated on a Spiral Concentrator (Amicon) with a S1Y100 membrane (100 kDa MW-CO). The concentrated broths are then applied to a Q Sepharose column (XK26, 120 ml) (Pharmacia, Uppsala, Sweden), preequilibrated with 10 mM Tris pH 7.5, 0.7 mS (Buffer A). Active fractions run through the column during loading and washing. The active fractions are pooled, adjusted to pH 5.3 and applied on a SP-Sepharose column (XK16, 60 ml) (Pharmacia, Uppsala, Sweden), preequilibrated with 10 mM MES pH 5.3 buffer (Buffer C). The majority of activity is eluted by a linear gradient of Buffer D (Buffer C and 1 mM NaCl). The active fractions are adjusted to 20 mS and applied to a Sephadex 200 column (1610, 120 ml) (Pharmacia, Uppsala, Sweden), pre-equilibrated with Buffer E (Buffer C and 0.1 M NaCl. Purified *Rhizoctonia solani* laccase fractions are eluted by Buffer E. A recovery of 1% or 5% and purification of 280- or 150-fold are achieved for mutants "M" and "T", respectively. The "T" mutant shows a three-fold higher yield than the "M" mutant, but two-fold lower yield than the wild-type laccase.

### Example 9: Characterization of the modified Myceliophthora thermophila laccases and the Rhizoctonia solani modified laccases

The Leu/Phe mutation causes a decrease in expression yield. RsL-"M" shows a yield which is three-fold lower than that of RsL-"T", and five-fold lower than that previously obtained for RsL-wild type; while MtL-"M" shows a yield approximately two-fold lower than that observed for MtL-"T" and five-fold lower than that observed for MtL-wild type. When *Polyporus pinsitus* laccase (PpL; U.S. patent No. 5,770,418), *Rhizoctonia solani* laccase (including isozyme 1 and 3; WO 95/07988), MtL, *Scytalidium thermophilum* laccase (StL), and *Myrothecium verrucaria* bilirubin oxidase (BiO) are expressed in the same host (HowB104), the yields are in the order of BiO ~ MtL ~ StL > RsL-4 > RsL-1 ~ PpL. Among these laccases, the residue corresponding to the modified Leu in the "M" mutants is: Phe for both PpL and RsL-1; Leu for RsL-4, MtL, and StL; and Met for BiO. It seems that a Phe at this particular position correlates to low expression yield of these laccases in *Aspergillus oryzae* HowB104 and HowB711 strains.

The triple mutations in RsL-mutant "T" (LEA - > VSG), which eliminates the negative charge, decreases activity two orders of magnitude. The triple mutations in MtL-mutant "T" (VSG - > LEA), which creates a negative charge, decreases activity 4-fold. In contrast, the "M" mutants, in which a Leu is replaced by a Phe, exhibit similar activity in comparison to their wild type counterparts. The *Rhizoctonia solani* results are consistent with the hypothesis which correlates the presence of negative charge(s) near the T1 Cu to the specific activity. The effect of the Glu in the selected pentapeptide segment could be attributed to general base-catalysis in which the negatively charged residue facilitates the electron transfer from the substrate to the T1 Cu by perturbing the substrate molecule and/or stabilizing the resulting electron-deficient intermediate or product molecule.

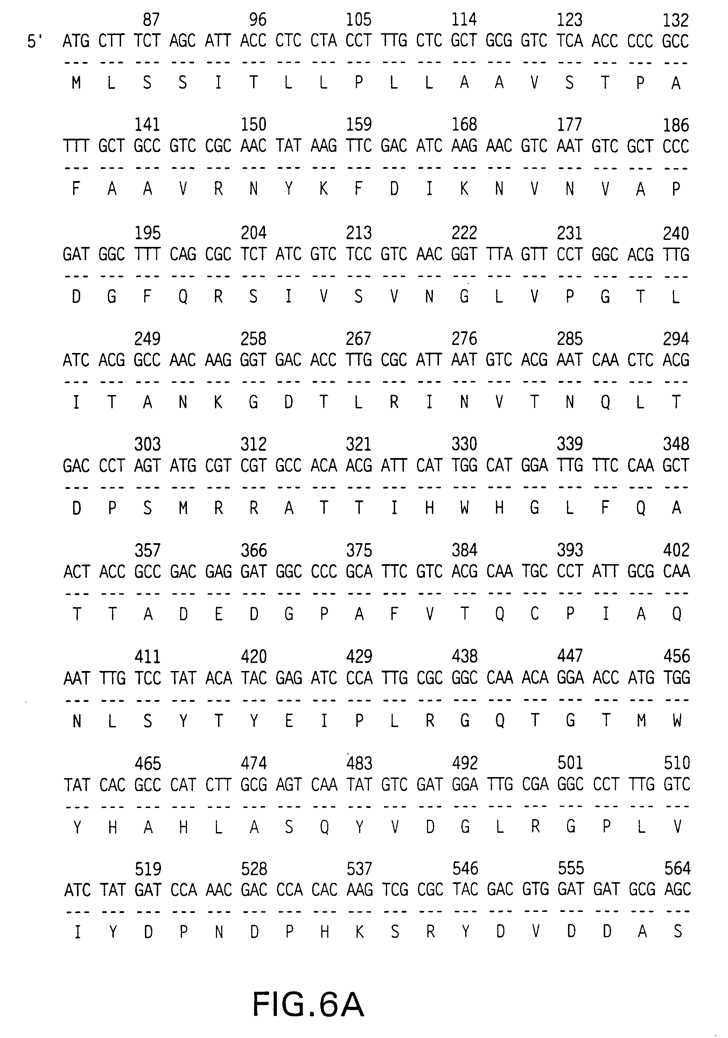

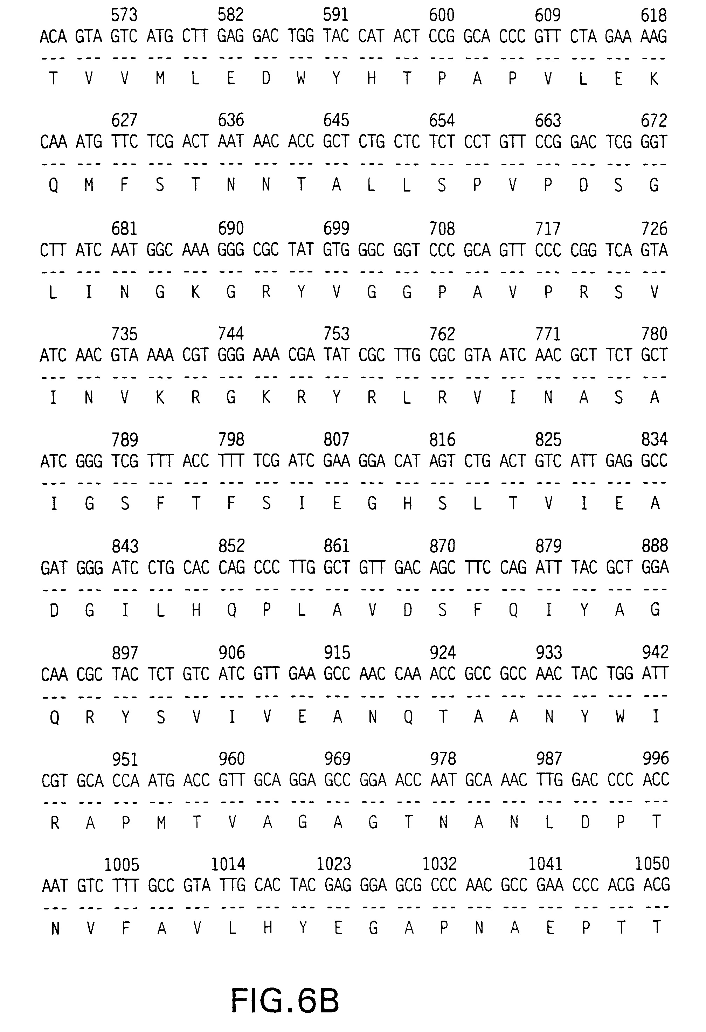

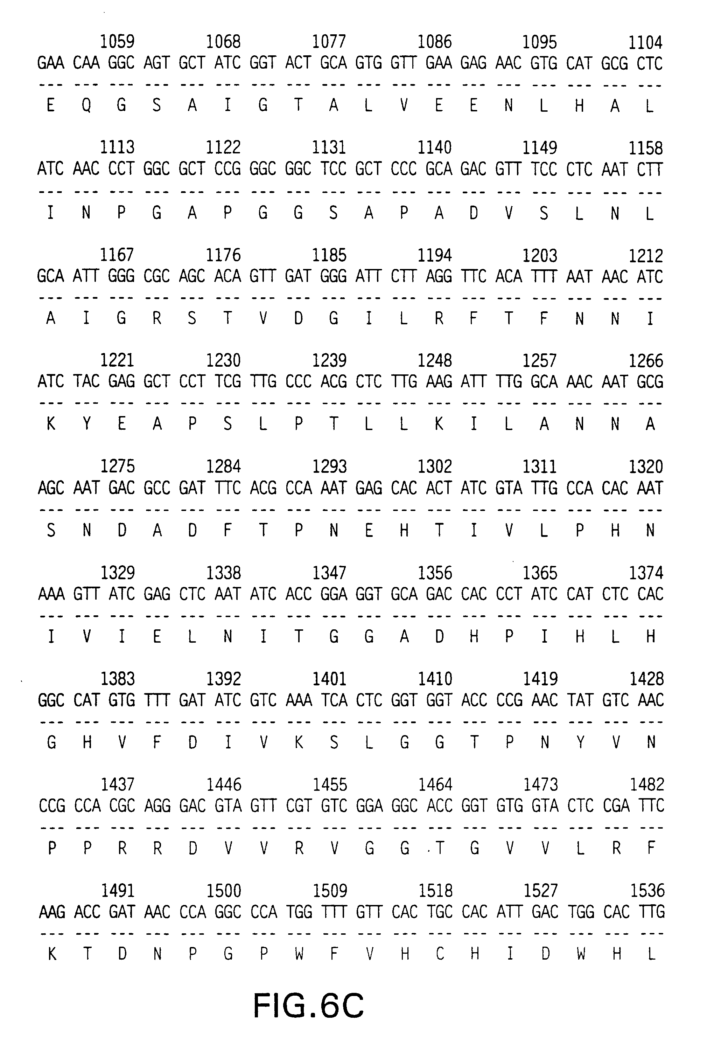

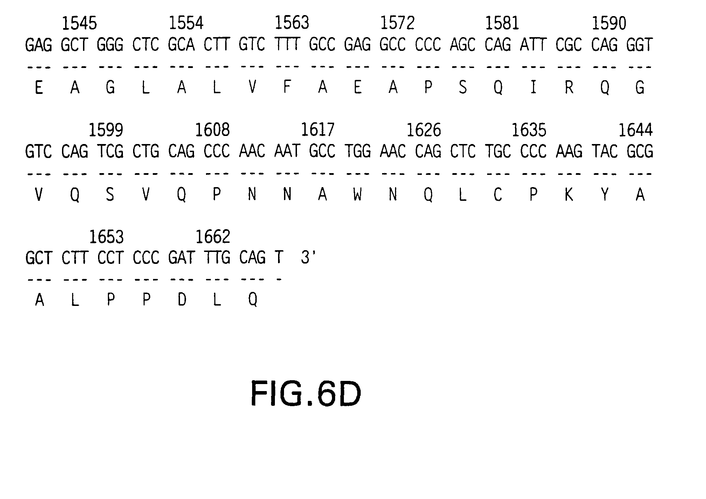

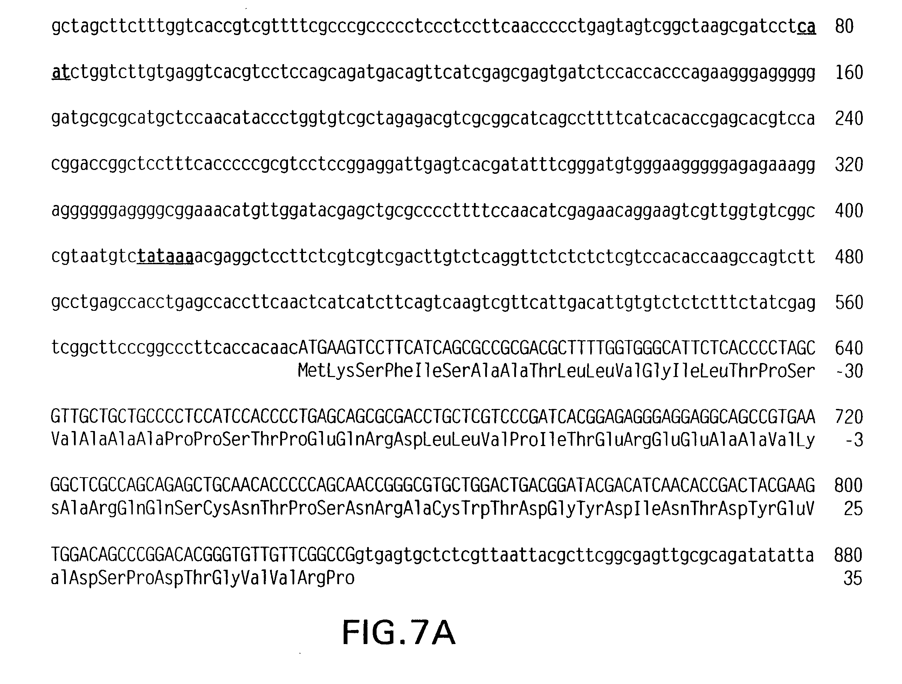

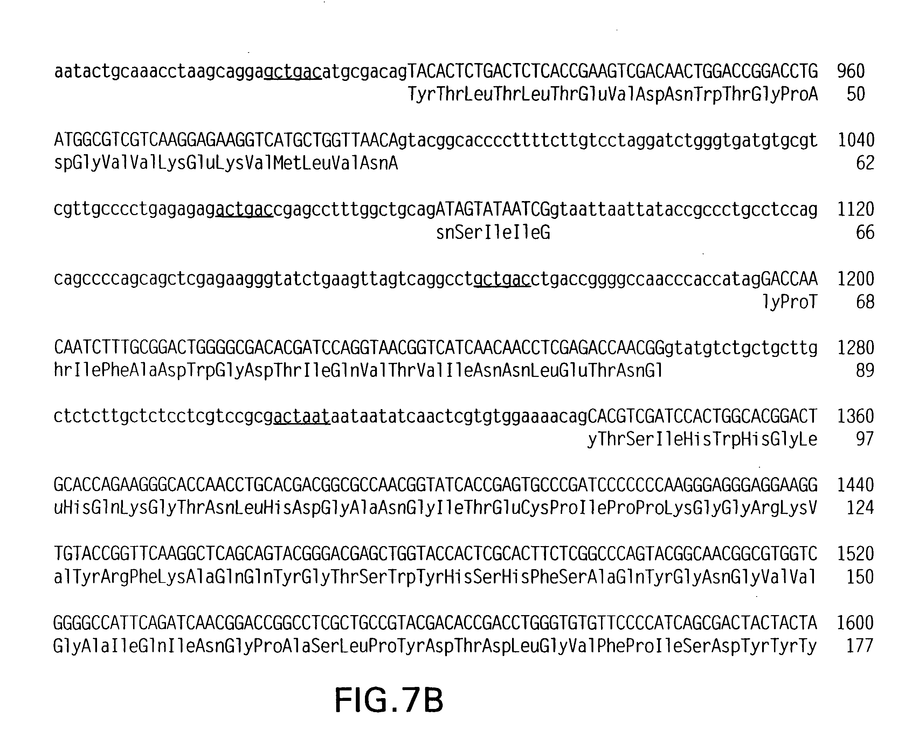

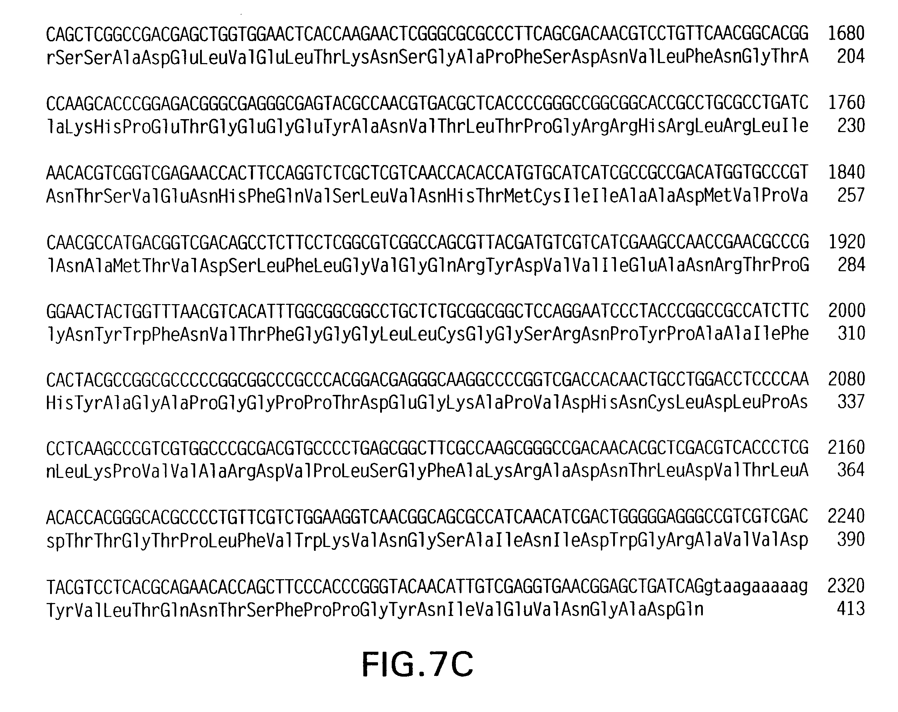

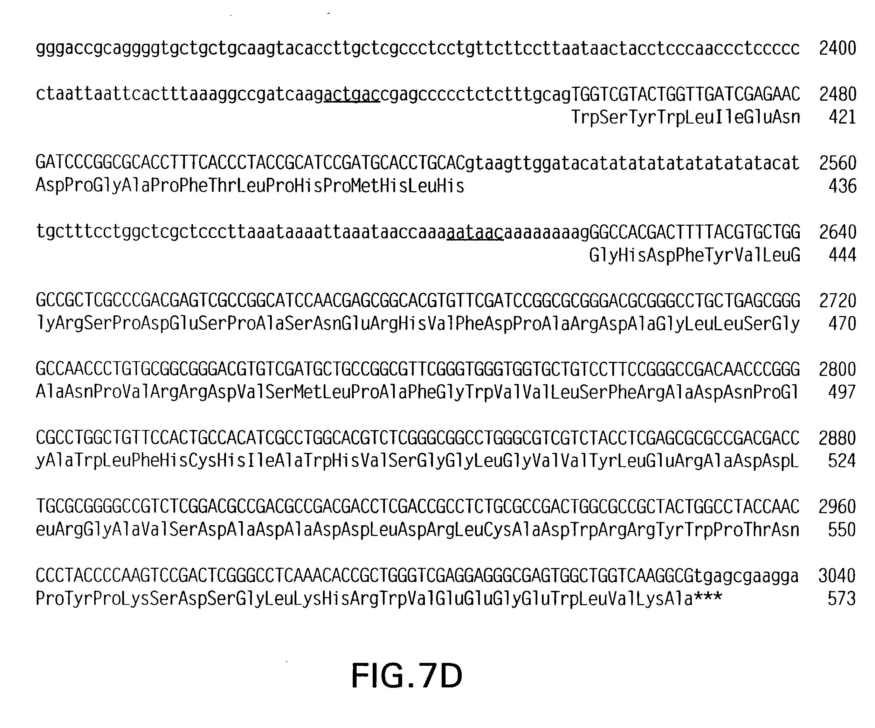

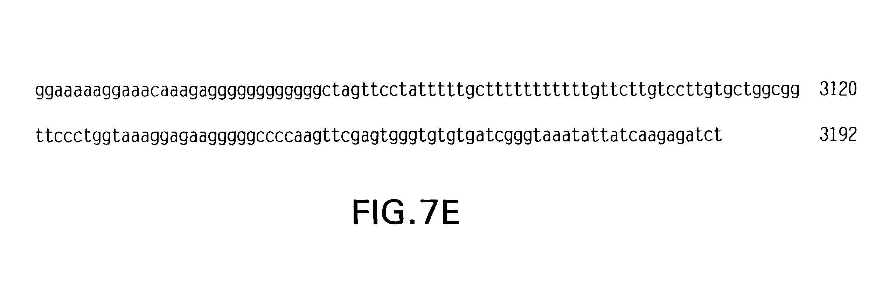

The molecular weights of the mature laccases are used to calculate both extinction coefficients and turnover numbers. The molecular weights are determined from the deduced amino acid sequences of the DNA sequences (Figure 6: SEQ ID NOS:24 and 25, and Figure 7: SEQ ID NOS:26 and 27). Protein concentrations (expressed as subunits) are measured based on the extinction coefficients determined by quantitative amino acid analysis.

Cyclic voltammetry measurements with a Pt electrode show a mid-potential of 0.76 V for Fe(dipyridyl)₂Cl₃ - Fe(dipyridyl)₂Cl₂ couple in 8 mM MES pH 5.3. The oxidation of ABTS and SGZ in 8 mM MES pH 5.3 yields a mid-potential of 0.70 and 0.63 V, respectively. The published redox potentials (E°) for the redox couples Fe(dipyridyl)₂Cl₃-Fe(dipyridyl)₂Cl₂, NaI₃ - NaI, and K₃Fe(CN)₆ - K₄Fe(CN)₆ are 0.780, 0.536, and 0.433 V, respectively (Kolthoff and Tomsicek, 1936, *Journal of Physical Chemistry* 40: 247-255; O'Reilly, 1973, *Biochimica Biophysica Acta* 292: 509-515; Vanysek, 1992, In Lide, D. R., editor, *Handbook of Chemistry and Physics,* 73rd Edition, pages 8.17-8.22, CRC Press, Boca Raton, Florida). The E° determination for *Rhizoctonia solani* laccase is performed in 8 mM MES pH 5.3 buffer with either 17 µM *Rhizoctonia solani* laccase, 0.2 mM Fe(bipyridyl)₂Cl₂, and 0.05 - 0.2 mM Fe(bipyridyl) ₂Cl₃, or 71 - 78 *µM Rhizoctonia solani* laccase and 14 - 100 µM ABTS. The E° determination for MtL is performed in 8 mM MES, pH 5.3 with 0.14 mM MtL, 0.02 - 20 mM K₃Fe(CN)₆, and 2 mM K₄Fe(CN)₆; as well as with 31 µM MtL, 0.1 mM I₂, and 5 - 19 mM NaI. Britten-Robinson buffer is used for other pHs. Under various potentials of the solution poised by various concentration ratios of the redox couples, the absorbance changes of laccase in the range of 550-800 nm are monitored and the concentrations of the oxidized copper (II) and reduced copper (I) states are calculated after the spectral change reaches equilibrium. The concentrations of the redox couples at equilibrium are calculated from the initial concentrations and the concentration changes caused by the interaction with laccase. In the case of measuring E° of *Rhizoctonia solani* laccase with ABTS, the concentration of ABTS cation radical (ABTS⁺) is measured by the absorption at 810 nm (where *Rhizoctonia solani* laccase has no contribution) and then the spectral contribution of ABTS⁺ at 600 nm is subtracted from the observed absorption value in order to assess the spectral change of *Rhizoctonia solani* laccase. Anaerobicity is achieved by repetitive evacuating and argon-flushing the reaction chamber at 4°C.

The mutants exhibit similar chromatographic elution patterns to their wild type counterparts. The purified preparations have a characteristic blue color typical of a laccase and show other typical laccase properties as shown in Table 4. All the mutants can be retained by a 100 kDa MW-CO membrane, indicating a dimeric nature.

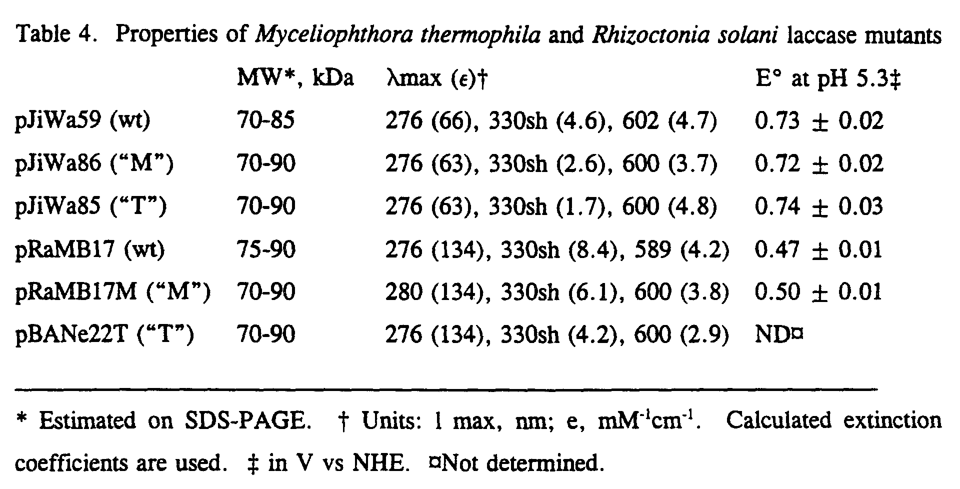

Kₘ and k_{cat} are obtained from the initial rate (v), enzyme concentration (E), and substrate concentration (S) in accordance to the equation v = k_{cat} ES/( Kₘ + S) by non-linear regression fitting using the Prizm program (GraphPad, San Diego, CA). The Kₘ and k_{cat} for ABTS and SGZ are measured spectroscopically in 8 mM MES-NaOH buffer, pH 5.3; while the values for other substrates are measured by oxygen electrode in Britten-Robinson buffer, pH 5.1 with a Hansatech DW1/AD device (Norfolk, England), with 0.4 - 4 µM laccase in 0.3 - 0.5 ml Britten-Robinson buffer. The O₂ concentration in air-saturated buffer solution is assumed as the same in plain water (0.28 mM).

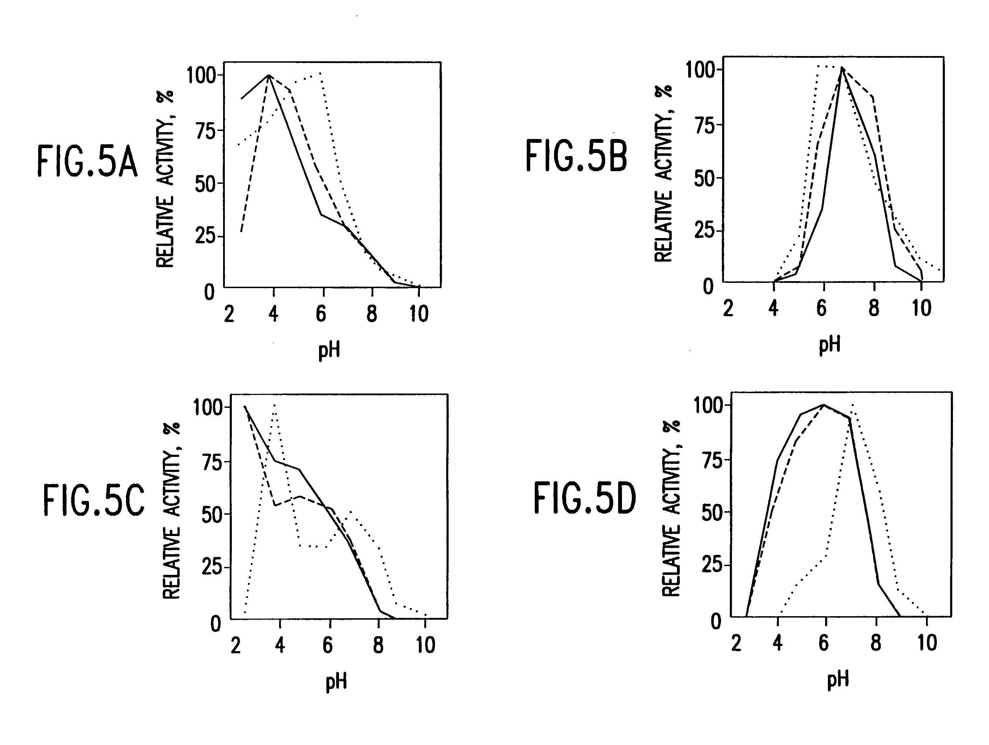

Tables 5 and 6 summarize the SGZ and ABTS oxidase activities of the mutants. For both *Rhizoctonia solani* laccase and *Myceliophthora thermophila* laccase, more profound difference is observed on the mutant "T" than that on the mutant "M" in comparison with the wild type. Figure 5 shows the pH-activity profiles of the mutants with ABTS and SGZ. For ABTS oxidation, a significant change is seen with RsL-"T", MtL-"M", and MtL-"T". The optimal pH of RsL-"T" is shifted ≥ 1 unit in comparison with the wild type laccase. For SGZ oxidation, an optimal pH at 7 is observed for MtL- T", in contrast to the range of 5-7 for the wild type laccase. In terms of pH profile, the elimination of the negative charge in RsL-"T" induces a shift of the optimal pH in the acidic direction for SGZ oxidation, probably due to the reduced acidity at the T1 site caused by the Glu removal. The creation of a negative charge in MtL-"T" induces a shift of the optimal pH for activity on the alkaline direction, which could be attributed to the increased acidity at MtL's T1 site caused by the creation of the negative charge.

**Table 5.**

| Syringaldazine oxidase activity of the mutants | | | |
|---|---|---|---|
| | LACU* | SOU† | (pHₒₚₜ) |
| RsL wt | 4.3 | 11 | (7) |
| RsL "M" | 2.2 | 4.7 | (6) |
| RsL "T" | 0.024 | 0.048 | (7) |
| MtL wt | 42 | 35 | (6) |
| MtL "M" | 24 | 25 | (6) |
| MtL "T" | 2 | 10 | (7) |
| Activity unit: µmol min⁻¹ mg⁻¹. *25 mM sodium acetate pH 5.5, 30°C. † B&R buffer, 20°C, at optimal pH (value in parenthesis). | | | |

**Table 6.**

| Syringaldazine and ABTS oxidase activity of the mutants | | | | |
|---|---|---|---|---|
| | SGZ | | ABTS | |
| | Kₘ, µM | k_{cat}, min⁻¹ | Kₘ, µM | k_{cat}, min⁻¹ |
| RsL wt | 28 ± 4 | 550 ± 40 | 52 ± 6 | 2500 ± 100 |
| RsL "M" | 35 ± 4 | 255 ± 11 | 125 ± 13 | 760 ± 30 |
| RsL "T" | 3.9 ± 0.3 | 1.1 ± 0.1 | 60 ± 4 | 20 ± 1 |
| MtL wt | 1.4 ± 0.2 | 4500 ± 200 | 110 ± 20 | 3800 ± 300 |
| MtL "M" | 1.8 ± 0.2 | 3300 ± 100 | 43 ± 3 | 1800 ± 100 |
| MtL: "T" | 0.9 ± 0.2 | 360 ± 20 | 11 ± 2 | 530 ± 20 |

## Claims

1. A modified wild-type *Rhizoctonia solani* laccase, which exhibits an altered pH activity profile relative to the wild-type *Rhizoctonia solani* laccase, comprising a mutation selected from the group consisting of (a) a substitution of one or more amino acid residues with other amino acid residues, (b) a deletion of one or more amino acid residues, as compared to the amino acid sequence of SEQ ID NO:25, wherein the amino acid sequence of SEQ ID NO:25 is modified in a segment of: 217INVKRGKRYR226, 303LDPTNVFAVL312, 356VSLNLAIGRSTVDGIL371, 416VIELNITGGADHPI429, or

474GPWFVHCHIDWHLEAGLALVF494.

2. A modified wild-type *Myceliophthora* laccase, which exhibits an altered pH activity profile relative to the wild-type *Myceliophthora* laccase, comprising a mutation selected from the group consisting of (a) a substitution of one or more amino acid residues with other amino acid residues, (b) an insertion of one or more amino acid residues and/or (c) a deletion of one or more amino acid residues, as compared to the wild-type *Myceliophthora* laccase, wherein the wild-type *Myceliophthora* laccase is modified in a segment corresponding to: 222GRRHRLRLIN231, 308AIFHYAGAPG317, 361VTLDTTGTPLFVWKVN376, 421ENDPGAPFTLPHPM433, or 497GAWLFHCHIAWHVSGGLGV515 of *Myceliophthora thermophila* laccase.

3. A laccase of claims 1 or 2 in which (a) a neutral amino acid residue is substituted with a negative charged amino acid residue or (b) a positive charged amino acid residue is substituted with a negative charged or neutral amino acid residue.

4. A laccase of claims 1 or 2 in which a phenylalanine is substituted with another amino acid residue.

5. A laccase of claim 4 in which the other amino acid residue is a leucine.

6. A laccase of claims 1 or 2 in which (a) a neutral amino acid residue is substituted with a positive charged amino acid residue or (b) a negative charged amino acid residue is substituted with a positive charged or neutral amino acid residue.

7. A laccase of claims 1 or 2 in which leucine or phenylalanine is substituted with a neutral residue selected from the group consisiting of histidine, serine, threonine, tyrosine, cysteine, and methionine.

8. A laccase of claims 1 or 2 which is modified by at least two amino acid residues.

9. A laccase of claim 8 which is modified by at least 3 amino acid residues.

10. A laccase of claims 1 or 2, wherein the mutation is a substitution.

11. A nucleic acid construct comprising a nucleic acid sequence encoding the laccase of claims 1 or 2.

12. A host cell comprising the construct of claim 11.

13. A method of altering the pH activity profile of a fungal laccase, comprising introducing a mutation selected from the group consisting of (a) a substitution of one or more amino acid residues with other amino acid residues, (b) an insertion of one or more amino acid residues and/or (c) a deletion of one or more amino acid residues, wherein the mutation is introduced in a segment corresponding to: 217INVKRGKRYR226, 303LDPTNVFAVL312, 356VSLNLAIGRSTVDGIL371, 416VIELNITGGADHPI429, 474GPWFVHCHIDWHLEAGLALVF494 of *Rhizoctonia solani* laccase, and 222GRRHRLRLIN231, 308AIFHYAGAPG317, 361VTLDTTGTPLFVWKVN376, 421ENDPGAPFTLPHPM433, 497GAWLFHCHIAWHVSGGLGV515 of *Myceliophthora thermophila* laccase.

## Patentansprüche

1. Modifizierte *Rhizoctonia-solani-Laccase* vom Wildtyp, die ein verändertes pH-Aktivitätsprofil gegenüber der *Rhizoctonia-solani-Laccase* vom Wildtyp aufweist, umfassend eine Mutation, ausgewählt aus der Gruppe, bestehend aus (a) einer Substitution von einem oder mehreren Aminosäureresten durch andere Aminosäurereste und (b) einer Deletion von einem oder mehreren Aminosäureresten verglichen mit der Aminosäuresequenz der SEQ ID NO: 25, wobei die Aminosäuresequenz SEQ ID NO: 25 in einem Segment von: 217INVKRGKRYR226, 303LDPTNVFAVL312, 356VSLNLAIGRSTVDGIL371, 416VIELNITGGADHPI429, oder 474GPWFVHGHIDWHLEAGLALVF494 modifiziert ist.

2. Modifizierte *Myceliophthora-Laccase* vom Wildtyp, die ein verändertes pH-Aktivitätsprofil gegenüber der *Myceliophthora-Laccase* vom Wildtyp aufweist, umfassend eine Mutation, ausgewählt aus der Gruppe bestehend aus (a) einer Substitution von einem oder mehreren Aminosäureresten durch andere Aminosäurereste, (b) einer Insertion von einem oder mehreren Aminosäureresten und/oder (c) einer Deletion von einem oder mehreren Aminosäureresten verglichen mit der *Myceliophthora-Laccase* vom Wildtyp, wobei die *Myceliophthora-Laccase* vom Wildtyp in einem Segment modifiziert ist, welches: 222GRRHRLRLIN231, 308AIFHYAGAPG317, 361VTLDTTGTPLFVWKVN376, 421ENDPGAPFTLPHPM433, oder 497GAWLFHCHIAWHVSGGLGV515 der *Myceliophthora-thermophila-Laccase* entspricht.

3. Laccase nach Anspruch 1 oder 2, in der (a) ein neutraler Aminosäurerest durch einen negativ geladenen Aminosäurerest ersetzt ist oder (b) ein positiv geladener Aminosäurerest durch einen negativ geladenen oder neutralen Aminosäurerest ersetzt ist.

4. Laccase nach Anspruch 1 oder 2, in der Phenylalanin durch einen anderen Aminosäurerest substituiert ist.

5. Laccase nach Anspruch 4, in der der andere Aminosäurerest Leucin ist.

6. Laccase nach Anspruch 1 oder 2, in der (a) ein neutraler Aminosäurerest durch einen positiv geladenen Aminosäurerest substituiert ist oder (b) ein negativ geladener Aminosäurerest durch einen positiv geladenen oder neutralen Aminosäurerest ersetzt ist.

7. Laccase nach Anspruch 1 oder 2, in der Leucin oder Phenylalanin durch einen neutralen Rest ersetzt ist, ausgewählt aus der Gruppe, die aus Histidin, Serin, Threonin, Tyrosin, Cystein und Methionin besteht.

8. Laccase nach Anspruch 1 oder 2, die durch mindestens zwei Aminosäurereste modifiziert wurde.

9. Laccase nach Anspruch 8, die durch mindestens drei Aminosäurereste modifiziert wurde.

10. Laccase nach Anspruch 1 oder 2, wobei die Mutation eine Substitution ist.

11. Nucleinsäurekonstrukt, umfassend eine Nucleinsäuresequenz, die die Laccase der Ansprüche 1 oder 2 kodiert.

12. Wirtszelle, umfassend ein Konstrukt gemäß Anspruch 11.

13. Verfahren zur Veränderung des pH-Aktivitätsprofils der Pilzlaccase, umfassend die Einführung einer Mutation, ausgewählt aus der Gruppe, die aus
(a) einer Substitution von einer oder mehreren Aminosäureresten durch andere Aminosäurereste, (b) einer Insertion von einem oder mehreren Aminosäureresten und/oder (c) einer Deletion von einem oder mehreren Aminosäureresten besteht, worin die Mutation in einem Segment eingeführt wurde, welches: 217INVKRGKRYR226, 303LDPTNVFAVL312, 356VSLNLAIGRSTVDGIL371, 416VIELNITGGADHPI429, 474GPWFVHCHIDWHLEAGLALVF494 der *Rhizoctonia-solani-Laccase* und 222GRRHRLRLIN231, 308AIFHYAGAPG317, 361VTLDTTGTPLFVWKVN376, 421ENDPGAPFTLPHPM433, 497GAWLFHCHIAWHVSGGLGV515 der *Myceliophthora-thermophila-Laccase* entspricht.

## Revendications

1. Laccase de *Rhizoctonia solani* de type sauvage modifiée, qui présente un profil d'activité de pH altéré par rapport à la laccase de *Rhizoctonia solani* de type sauvage, comportant une mutation sélectionnée parmi le groupe constitué de (a) une substitution d'un ou plusieurs résidus d'acide aminé par d'autres résidus d'acide aminé, (b) une délétion d'un ou plusieurs résidus d'acide aminé, par comparaison à la séquence d'acides aminés de la SEQ ID N° :25, dans laquelle la séquence d'acides aminés de la SEQ ID N° :25 est modifiée dans un segment de : 217INVKRGKRYR226, 303LDPTNVFAVL312, 356VSLNLAIGRSTVDGIL371, 416VIELNITGGADHPI429, or

474GPWFVHCHIDWHLEAGLALVF494.

2. Laccase de *Myceliophthora* de type sauvage modifiée, qui présente un profil d'activité de pH altéré par rapport à la laccase de *Myceliophthora* de type sauvage, comportant une mutation sélectionnée parmi le groupe constitué de (a) une substitution d'un ou plusieurs résidus d'acide aminé par d'autres résidus d'acide aminé, (b) une insertion d'un ou plusieurs résidus d'acide aminé et/ou (c) une délétion d'un ou plusieurs .résidus d'acide aminé, par comparaison à la laccase de *Myceliophthora* de type sauvage, dans laquelle la laccase de *Myceliophthora* de type sauvage est modifiée dans un segment correspondant à : 222GRRHRLRLIN231, 308AIFHYAGAPG317, 361VTLDTTGTPLFVWKVN376, 421ENDPGAPFTLPHPM433, ou 497GAWLFHCHIAWHVSGGLGV515 d'une laccase de *Myceliophthora thermophila.*

3. Laccase selon la revendication 1 ou 2, dans laquelle (a) un résidu d'acide aminé neutre est substitué par un résidu d'acide aminé chargé négatif ou (b) un résidu d'acide aminé chargé positif est substitué par un résidu d'acide aminé neutre ou chargé négatif.

4. Laccase selon la revendication 1 ou 2, dans laquelle une phénylalanine est substituée par un autre résidu d'acide aminé.

5. Laccase selon la revendication 4, dans laquelle l'autre résidu d'acide aminé est une leucine.

6. Laccase selon la revendication 1 ou 2, dans laquelle (a) un résidu d'acide aminé neutre est substitué par un résidu d'acide aminé chargé positif ou (b) un résidu d'acide aminé chargé négatif est substitué par un résidu d'acide aminé neutre ou chargé positif.

7. Laccase selon la revendication 1 ou 2, dans laquelle une leucine ou phénylalanine est substituée par un résidu neutre sélectionné parmi le groupe constitué d'histidine, sérine, thréonine, tyrosine, cystéine, et méthionine.

8. Laccase selon la revendication 1 ou 2, qui est modifiée par au moins deux résidus d'acide aminé.

9. Laccase selon la revendication 8, qui est modifiée par au moins 3 résidus d'acide aminé.

10. Laccase selon la revendication 1 ou 2, dans laquelle la mutation est une substitution.

11. Produit de synthèse d'acide nucléique comportant une séquence d'acide nucléique codant pour la laccase selon la revendication 1 ou 2.

12. Cellule hôte comportant le produit de synthèse selon la revendication 11.

13. Procédé pour altérer le profil d'activité de pH d'une laccase fongique, comportant l'introduction d'une mutation sélectionnée parmi le groupe constitué de (a) une substitution d'un ou plusieurs résidus d'acide aminé par d'autres résidus d'acide aminé, (b) une insertion d'un ou plusieurs résidus d'acide aminé et/ou (c) une délétion d'un ou plusieurs résidus d'acide aminé, dans lequel la mutation est introduite dans un segment correspondant à : 217INVKRGKRYR226, 303LDPTNVFAVL312, 356VSLNLAIGRSTVDGIL371, 416VIELNITGGADHPI429, 474GPWFVHCHIDWHLEAGLALVF494 d'une laccase de *Rhizoctonia solani,* et 222GRRHRLRLIN231, 308AIFHYAGAPG317, 361VTLDTTGTPLFVWKVN376, 421ENDPGAPFTLPHPM433, 497GAWLFHCHIAWHVSGGLGV515 d'une laccase de *Myceliophthora thermophila.*
